Europäisches Patentamt

European Patent Office

Office européen des brevets

(11) Publication number: 0 426 933 A1

# EUROPEAN PATENT APPLICATION

(21) Application number: 90102653.4

(22) Date of filing: 10.02.90

(51) Int. Cl.5: C02F 3/30, C02F 9/00

The title of the invention has been amended (Guidelines for Examination in the EPO, A-III, 7.3).

(30) Priority: 28.09.89 IT 2185889

(43) Date of publication of application:
15.05.91 Bulletin 91/20

(84) Designated Contracting States:
AT BE CH DE DK ES FR GB GR IT LI LU NL SE

(71) Applicant: FOSTER WHEELER ITALIANA S.p.A.
Via S. Caboto 1
I-20094 Corsico, Milan(IT)

(72) Inventor: Della Sala, Umberto
Viale Libertà 17
Pavia(IT)
Inventor: Fava, Andrea
V.1e Damiano Chiesa 5
Rivanazzano, Pavia(IT)
Inventor: Bianco, Vito
Via Forze Armate 260/3
Milan(IT)

(74) Representative: Klausner, Erich et al
c/o Ufficio Internazionale Brevetti Ing. C.
Gregorj S.p.A. Via Dogana 1
I-20123 Milano(IT)

(54) Method and plant for the treatment of leachate from sanitary landfills for municipal solid waste and similar wastes.

(57) The present invention relates to a method and plant for the depuration of leachate from municipal solid waste landfills or of comparably similar waste liquors. The waste liquor is subjected to an anaerobic treatment with production of biogas and precipitation of heavy metals. An anoxic denitrification step is then carried out, followed by a biological oxidation-nitrification step in which the removal of the organic pollutant components present in the percolate is completed, and the almost complete oxidation of ammonia to nitrate ion for the subsequent reduction in anoxic conditions of the nitrate ion to nitrogen is assured.

If so required by the limits imposed on the effluent, the effectiveness of removal of the organic substances contained in the percolate can be enhanced by the addition of suitably proportioned amounts of powdered activated carbon into the biological oxidation reactor or by means of a filtration of the effluent on granular activated carbon.

## A METHOD FOR THE TREATMENT OF LEACHATE PRODUCED BY THE SANITARY LANDFILLS FOR MUNICIPAL SOLID WASTE AND SIMILAR WASTES AND PLANT FOR CARRYING OUT SAME

The present invention relates to a method and plant for the depuration of leachate from municipal solid waste and similar wastes.

The method can also be used for the depuration of waste liquors with a high content of organic and nitrogen pollutants.

The percolate from the controlled dumps for municipal solid waste and similar wastes is a liquor characterized by a high degree of pollution, mainly due to the presence of a high concentration of organic substances, ammonia nitrogen and heavy metals.

The percolate has been up to now disposed of either by transferring the liquor to an external sewage treatment plant or by means of treatment at landfill site, said treatment being mainly based on chemical-physical processes (clariflocculation with lime) or on aerobic biological processes (ponds, activated sludge).

All the employed solutions have shown a series of limitations and problems, both from the technical and from the economical point of view, pointing out to the need for the research and development of new method.

In particular, besides the non-negligible economic and logistic aspects (cost of transportation, disposal, and so on), the complexity and very high variability of the analytical composition and of the amount of percolate give rise to a series of technical problems in the combined treatment of leachate with urban sewage liquor in an existing depuration plant.

Said problems are mainly related with the eccessive organic and ammonia load to the biological unit, the influence of heavy metals onto the bacterial metabolism, and the alteration of resulting sludge quality.

The physical-chemical and aerobic biological processes, alone or combined, have a series of limits as well, said limits arising from the remarkable energy consumption and space needs (aerobic biological processes) and from the high consumption of chemicals and high production of residual chemical sludges (chemical-physical processes).

The object of the present invention is to provide a method by means of which the above-mentioned drawbacks can be overcome and that can be implemented in practice with the use of high steadiness and efficiency, low cost biological processes.

The method according to the invention, that is based on an anaerobic treatment of the liquor followed by a two stage biological denitrification-nitrification process of the activated sludge type, has the following essential advantages.

Compared with the conventional techniques, most (80-90%) of the pollutant load of an organic nature is removed during the anaerobic processes with an energy consumption of about 1/10 of that of aerobic processes of similar efficiency, thereby obtaining as a by-product a combustible gas (biogas) that is in a measure of balancing the thermal energy required by the anaerobic reactor.

The excess biological sludge is similarly reduced in a degree of about 1/5 - 1/6, thereby obtaining, moreover, a sludge with a higher dry substance contents and better dewatering characteristics, the anaerobic process assuring at the same time an effective reduction of the heavy metal contents, that are toxic to the aerobic biological processes.

The anaerobic pretreatment finally allows the performance of the downstream aerobic processes to be best exploited, in that said aerobic processes operate in the optimal conditions of pollutant load for the use of this kind of processes.

As better described hereinafter, for the percolate anaerobic treatment step, among the various available technological solutions, a completely mixed reactor is preferably employed, said system being especially suitable for the subject liquors, especially as far as the managing ease, operational relyability and low running costs are concerned.

The next step of the denitrification-nitrification biological treatment comprises an anoxic denitrification followed by a biological oxidation-nitrification step.

The oxidation step is carried out in a completely mixed reactor in which, by maintaining suitable operation conditions (organic load, sludge age, and so on) the residual organic contents of the liquor can be degraded and the organic and ammonia nitrogen can be almost completely nitrified.

The biological denitrification of the nitrates is carried out in a denitrification reactor upstream of the nitrification reactor, a suitable proportion of nitrate-rich liquor, that is taken from the aerobic reactor, being circulated back to said denitrification reactor.

The optimum $TOC/N-NO_3$ ratios (wherein TOC stands for total organio carbon) that are needed for the complete reduction of the nitrate ions to nitrogen are maintained by feeding to the denitrification step an aliquot of the raw leachate, said leachate having being previously subjected to a chemical treatment for the precipitation of the heavy metals, that are toxic to the biological processes.

All the produced biological sludge (a quite low amount, actually) from the processes is recycled to the anaerobic reactor where it is "digested" and it is then passed to the dewatering step and final disposal.

It is known that the leachate from the municipal solid waste landfills is characterized by a widely variable composition depending on the meteorological conditions, the age of the waste dump and the way it is runned.

The depuration method of the present invention can be applied within a wide range of variation of the leachate characteristics, said range being illustratively shown in the following table:

| | |
|---|---|
| COD mg/l | 1,000 - 60,000 |
| $BOD_5$ mg/l | 800 - 25,000 |
| SS mg/l | 300 - 1,500 |
| VSS mg/l | 100 - 750 |
| TKN mg/l | 600 - 2,600 |
| $N-NH_4$ mg/l | 500 - 2,200 |
| pH | 6 - 9 |
| Volatile acids mg/l | 1,000 - 15,000 |
| Fe mg/l | 30 - 300 |
| Zn mg/l | 0.8 - 10 |
| Cd mg/l | 0.001 - 0.007 |
| $Cr_{tot}$ mg/l | 0.07 - 0.4 |
| Ni mg/l | 0.06 - 0.6 |
| Cu | 0.04 max |
| Pb | 0.01 - 0.05 |

The above abbreviations having the following meaning:

COD = chemical oxigen demand

$BOD_5$ = biochemical oxigen demand

S S = suspended solids

V S S = volatile suspended solids

TKN = total nitrogen, as measured according to the Kijeldhal method.

The illustrative plant is designed for obtaining an effluent generally within the limits imposed by the regulations of the various Countries.

If the plant of the present invention is operated with a percolate having COD concentrations close to the upper limits of the above-mentioned ranges, the lower COD limits in the treated effluent, for example 500 mg/l, are achieved by proportioning powdered activated carbon directly into the aerobic oxidation step, said activated carbon having suitable surface area and physical characteristics.

As an alternative, the same results can be obtained by filtrating the effluent on columns packed with granular activated carbon. The use of activated carbon, either in granular or in powder form, is not required in case the so called "polishing" or final depuration of the effluent in an external municipal treatment plant can be carried out.

The present invention does not provide for the removal of chlorides or inorganic salts in general, that are not removed by the biological processes and for which there is no economical reason of using specific removal technologies.

The salt contents of the leachate (ca. 2,000 mg/l of Cl) are however not so high as to impair the efficiency of the biological processes.

The leachate treatment plant essentially comprises an anaerobic treatment section and a final denitrification/nitrification step.

The main section of the plant comprises an aerobic reactor; the anaerobic treatment allows in fact, with a very low energy consumption, high ratios of organic pollutants to be converted into a combustible gas (biogas), said biogas being easily used for a possible energy recovery. The leachate, as it is extracted from the landfill, is especially rich of volatile fatty acids and it is therefore an ideal substrate for the methane producing reactions, that is for the conversion reactions of the fatty acids into methane, that are the final step of the biological degradation processes of the organic substances by the anaerobic bacteria.

A further advantage of the anaerobic process is that it can eliminate the heavy metals present in the

3

leachate.

In fact, the biological reduction of the sulphates to hydrogen sulphide in anaerobic conditions provides an effective precipitating agent for most of the heavy metals that are present in the percolate of a sanitary landfill.

An aiding effect in the precipitation of the metals is provided by the increased alkalinity produced during the process of anaerobic degradation of the organic substrates, said increased alkalinity allowing a part of the metal to be precipitated in the form of carbonates, hydroxides and/or organo-metallic compounds.

The anaerobic treatment is therefore also an efficient protecting step for the aerobic section downstream, that otherwise might be subject to chronic toxicity troubles due to the accumulation of heavy metals in the biological sludge. The anaerobic reactor also provides for efficiently levelling out the peaks of COD/BOD$_5$ concentration, assuring the biological system downstream to be regularly and steadily supplied.

The type of anaerobic reactor that is preferably employed is the mesophilic completely mixed reactor. In such a reactor, the biomass and organic substrate concentrations are kept almost constant by means of a suitable, low energy input mixing system.

In the present embodiment, the completely mixed configuration of the anaerobic reactor offers the advantage that the reactor can be supplied directly with the raw liquor, with no previous removal of the suspended solids, that would negatively affect other possible technological alternatives for the anaerobic reactor (UASB, fluidized bed, and so on).

The reactor also comprises an external sludge separator for the separation of the biomass from the treated water, and the related system for recycling the biomass to the reactor.

The optimum temperature for carrying out the organic substrate degradation reactions by the bacterial mass is kept by pre-heating the reactor feed in a heat exchanger and by providing the apparatus with a suitable insulating jacket.

No heat recovery is provided on the liquor from the anaerobic reactor, thereby rising the operating temperature of the biological denitrification-nitrification system to the values (30-32°C) at which the reaction kinetics are fastest.

The biological denitrification/nitrification system downstream of the anaerobic step is designed to assure an almost complete oxidation of ammonia and organic nitrogen to nitrate, followed by a reduction in anoxic condition of nitrates to nitrogen.

The high nitrification and denitrification efficiencies required are obtained by operating the oxidative step at a low organic load (that is high sludge age) and by keeping optimum TOC/N-NO$_3$ ratios in the feed to the denitrification system.

To this purpose, a portion of the raw percolate is directly fed to the denitrification reactor after it has undergone a treatment for the chemical precipitation of the heavy metals, that are toxic to the biological processes. For the start-up of the denitrification section and for facing emergency situations, the possibility of proportioning to the denitrification reactor an external supply of T.O.C. (methanol, glucose, sand so on) is provided.

Depending on the leachate characteristics and/or the discharge limits, it may be necessary to enhance the efficiency of removal of the organic pollutants contained in the leachate; this can be achieved by adding powdered activated carbon into the biological oxidation section or by filtering the effluent on columns packed with granular activated carbon.

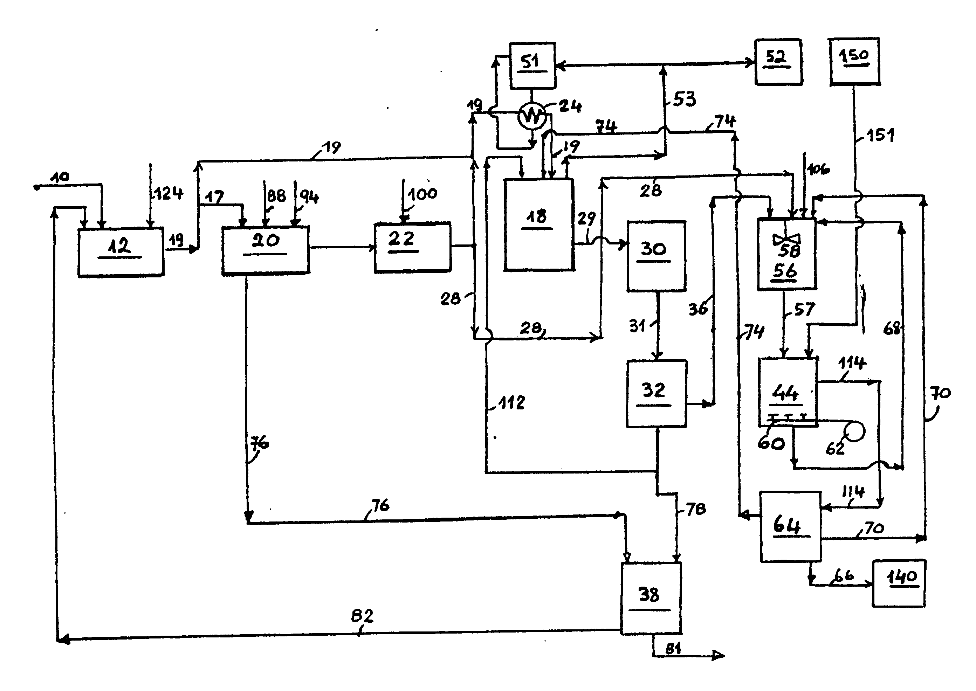

The invention will be described hereinafter with reference to the enclosed drawing in which the parts of a plant adapted to carry out the depuration method according to the invention are diagrammatically shown.

The raw waste liquor to be treated, supplied through line 10, is collected in an equalization reservoir or tank 12, the content of which is suitably mixed to the purpose of homogenizing its characteristics, and transferred to the anaerobic reactor 18 through line 19. An aliquot of the homogenized leachate is sent via lines 19 and 17 to an apparatus 20 for the precipitation of the heavy metals, said apparatus possibly consisting of a clari-flocculator, a flocculator and a settler or other suitable apparatus, in said apparatus 20 an alkali through line 88 and a coagulating agent through line 94 being proportioned to the purpose of precipitating the heavy metals and separating them together with the suspended solids that are contained in the waste liquor. After correcting the pH value with acid in the neutralization apparatus 22, the waste liquor is supplied through line 100, in the suitable ratio, to the denitrification reactor 56, partly by-passing the anaerobic reactor through line 28.

The acid needed for keeping a suitable pH value during the anaerobic step is proportioned into tank 22 directly downstream of the equalization through line 100, while the stream fed to the anaerobic reactor 18 is brought to the optimum temperature values in the pre-heating apparatus 24. The biogas that is produced in reactor 18 is transferred through line 53 to recovery 51 or, in emergency, exhausted to flare 52.

The by-pass line 28 of the anaerobic section allows the organic load sent to denitrification tank 56 to be so controlled as to keep in the latter the proper TOC/N-NO₃ ratios.

The effluent from the anaerobic reactor 18 passes through line 29 to a degassing unit 30, to remove the gas bubbles that it might have entrapped, and gravity flows to sludge separator 32, that may comprise a plate separator, a sedimentation basin, a decanter centrifuge or other apparatus suitable for separating the treated liquor from the biological sludge; the waste liquor is then sent to the final denitrification/nitrification step 56, 44 through line 36, the sludge being recycled through line 112 to the anaerobic reactor 18. The excess sludge from separator 32 is transferred to the thickener 38 through line 78.

The effluent that has been clarified in the sludge separator 32 is transferred through line 36 to the biological denitrification reactor 56, said reactor 56 possibly comprising a closed tank, a pond, a concrete basin, a reservoir or other suitable apparatus, in which it is mixed with the ricycle stream from the subsequent nitrification reactor 44 through line 68. The mixing system 58 of reactor 56 provides for a thorough homogenization of the reactor contents without giving rise to an oxigenation of the mixture, thus safeguarding the anoxic conditions that are needed for the denitrification. To reactor 56 an external organic carbon supply can be fed through line 106.

The activated sludge that is contained in the denitrification reactor 56 flows by gravity through line 57 to the biological nitrification reactor 44, said reactor 44 comprising a closed tank, a lagoon or cement basin, or other suitable apparatus and being equipped with air spargers 60 and related blowers 62 or with similar oxygen transfer systems so as to assure the oxygen supply that is needed for the oxidation of the residual BOD and the nitrification of nitrogen.

To reactor 44, powdered activated carbon can be supplied from proportioning apparatus 150 through line 151.

sThe effluent from the nitrification reactor 44 finally passes through line 114 to the final decanter 64, the clarified liquor from which is transferred through line 66 to its final destination or to the final filtration unit 140, where it is filtered on granular activated carbon; the bottom sludge is sent to denitrification step 56, through line 70. The overflow sludge is periodically transferred to the anaerobic reactor 18 through line 74.

The plant is completed by a sludge thickener 38 that receives the chemical sludges from precipitator 20 through line 76 and the excess biological sludges from sludge separator 32 through line 78; the thickened sludge is removed through line 81 to a sludge dewatering unit and the surnatant is transferred to equalization 12 through line 82. To equalization 12 a phosphorous supply is fed through line 124.

## Claims

1. A method for the treatment of the leachate from municipal solid waste landfills and similar waste waters, characterized by the following operation steps:

a) anaerobic treatment of the waste liquor for converting, with a minimum energy supply, a high percentage of organic pollutants into biogas and

b) contemporaneous precipitation of the heavy metals;

c) anoxic biological denitriflcation of the leachate;

d) biological nitrification-oxidation, possibly enhanced with powdered activated carbon to complete the removal of the organic pollutants still present in the leachate and oxidize ammonia and organic nitrogen to nitrate; the thus produced nitrates being recycled in the suitable ratios to the anoxic biological denitrification to be reduced to nitrogen;

e) supply of a part of the raw leachate to the denitrification step, after precipitation of the heavy metals, to the purpose of maintaining the optimum TOC/N-NO₃ ratios;

f) possibly filtration of the final effluent on granular activated carbon.

2. A method according to claim 1, characterized in that the waste liquor, before reaching the anaerobic reactor, passes through an equalization step to the purpose of levelling out its characteristics, also with the supply of a proportioned amount of phosphorus, and is preheated to bring it to the optimum temperature values.

3. A method according to claim 1 and 2, characterized in that a part of homogenized waste liquor is treated by adding an alkali and a coagulating agent to precipitate by sedimentation the heavy metals together with the suspended solid materials, and then, after correcting the pH value by means of an acid, it is supplied under suitable ratios to a denitrification treatment, by-passing the anaerobic reactor.

4. A method according to claims 1 to 3, characterized in that from the anaerobic reactor, in which the biomass and organic substrate concentrations are kept almost uniform by means of a suitable, low energy input mixing system, combustible gas is recovered for thermal uses, such as for example the pre-heating of the leachate, the effluent of the reactor, after a further degasification, being separated from the biological sludge and transferred to the denitrification reactor together with the homogenized waste liquor from which the suspended solid and heavy metals have been precipitated.

5. A method according to claims 1 to 4, characterized in that the biological sludge separated from the effluent of the anaerobic reactor is partly recycled to the same reactor and partly transferred to final disposal through a thickening step.

6. A method according to claims 1 to 5, characterized in that the leachate downstream of the denitrification step is subjected to a subsequent nitrification in a biological nitrification-oxidation reactor into which oxygen is supplied, then it is subjected to a sedimentation step for separating the clarified surnatant to be sent to the final destination or to a further filtration over granular activated carbon, the bottom sludge being recycled to the denitrification and the excess sludge being periodically recycled to the anaerobic reactor.

7. A plant for carrying out the method for the treatment of percolate from municipal solid waste landfills according to claims 1 to 6, characterized by an anaerobic reactor (18) for the production of biogas, said anaerobic reactor being connected upstream through line (19) to an equalization tank (12) into which the percolate is introduced, said tank being connected in series with apparatuses (20 and 22) for the precipitation of the heavy metals and the neutralization of leachate, respectively; the reactor (18) being connected downstream to a degassing unit (30) through line (29), the degassing unit being connected to a sludge separator (32) through line (31) and the separator (32) being connected to a sludge thickening unit (38) through line (78), said plant comprising sidewise a further series of apparatuses (56, 44, 64) partly connected to said members, said apparatuses being respectively provided for the denitrification, nitrification and final sedimentation of the leachate.

8. A plant according to claim 7, characterized in that the equalization tank (12) is a terminal of line (124) for the supply of phosphorus, said tank being connected through line (17) to apparatus (20), said apparatus (20) being a terminal of lines (88) and (94) for the supply of an alkali and a coagulating agent, respectively, said apparatus (20) being connected to the neutralization apparatus (22) that is supplied with an acid through line (100).

9. A plant according to claims 7 and 8, characterized in that said neutralization apparatus (22) is connected downstream through line (28) to the denitrification apparatus (56), said line (28) possibly joining also line (19) before the latter passes through a pre-heater (24) that is suitable to bring the leachate to the optimum temperature for the anaerobic treatment that is carried out in reactor (18).

10. A plant according to claims 7 to 9, characterized in that the sludge thickener (38) is connected through line (76) to the apparatus (20) for the precipitation of the heavy metals and through line (78) to the separator (32) from which it receives the sludges, said thickened sludge being removed from said thickener through line (81) and the surnatant being recycled to the equalization tank (12) through line (82).

11. A plant according to claim 7 to 10, characterized in that a line (112) for the sludge recycle is provided between the discharge of sludge separator (32) and the anaerobic reactor (18).

12. A plant according to claims 7 to 11, characterized in that the denitrificator (56) is connected to sludge separator (32), from which it receives the waste liquor, through line (36), to the final sedimentation unit (64) through line (70) for recycling the bottom sludge and to the nitrification reactor (44) through line (68) for recycling the activated sludge slurry, said denitrificator possibly receiving a supply of organic carbon through line (106).

13. A plant according to claims 7 to 12, characterized in that the contents of denitrificator (56) are transferred through line (57) to the nitrification/oxidation reactor (44), said nitrification/oxidation reactor (44) being provided with air spargers (60) and related blowers (62) or similar systems for the transfer of oxygen to assure the necessary supply of oxygen to said reactor (44), the effluent of said tank (44) being transferred to the final sedimentation unit (64) through line (114).

14. A plant according to claims 7 to 13, characterized in that the nitrification tank (44) that is connected downstream, through line (114), to the final sedimentation unit (64) receives, through line (151), powdered activated carbon frog a proportioning unit (150).

15. A plant according to claims 7 to 14, characterized in that the excess biological sludge is transferred from sedimentation unit (64) to anaerobic reactor (18) through line (74).

16. A plant according to claims 7 to 15, characterized in that the final sedimentation unit (64) is connected through line (66) to an assembly (140) for the filtration on granular activated carbon.

17. A plant according to claims 7 to 16, characterized in that the biogas that is produced in the anaerobic reactor (18) is transferred to the recovery unit (51) through line (53) or, in case of emergency, exhausted to a flare (52) provided in said plant.

18. A plant according to claim 7, characterized in that the anaerobic reactor (18) can be a mesophilic, completely mixed reactor type in which the concentrations of biomass and organic substrate are kept almost uniform through the reactor by means of a suitable, low energy input mixing system.

European
Patent Office

**EUROPEAN SEARCH
REPORT**

Application Number

**EP 90 10 2653**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,P | DATABASE WPIL, accesion no. 90-088608 [12], Derwent Publications Ltd, London, GB; & NL-A-88 01 995 (GRONTMY) * Claims; figure * | 1,2,4 | C 02 F 3/30 C 02 F 9/00 |
| A | DE-A-3 833 185 (LINDE AG) * Abstract * | 1 | |
| A | EP-A-0 051 888 (GIST BROCADES) * Figure 2 * | 1 | |
| A | US-A-4 366 059 (WITT et al.) * Abstract * | 1 | |

TECHNICAL FIELDS
SEARCHED (Int. Cl.5)

C 02 F

The present search report has been drawn up for all claims

| Place of search | Date of completion of search | Examiner |
|---|---|---|
| The Hague | 03 January 91 | KASPERS H.M.C. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document